Europäisches Patentamt

European Patent Office

Office européen des brevets

(11) Publication number: **0 174 999 B1**

(12) **EUROPEAN PATENT SPECIFICATION**
published in accordance with Art.
158(3) EPC

(45) Date of publication of patent specification: **17.04.91** (51) Int. Cl.⁵: **C12N 15/00, C07H 21/04, C12P 21/02, A61K 37/66**

(21) Application number: **85902003.4**

(22) Date of filing: **16.03.85**

(86) International application number:
**PCT/EP85/00109**

(87) International publication number:
**WO 85/04186 (26.09.85 85/21)**

Divisional application 88119117.5 filed on
16/03/85.

(54) Method for improving expression and method thereto.

(30) Priority: **16.03.84 GB 8406910**
**24.05.84 GB 8413297**

(43) Date of publication of application:
**26.03.86 Bulletin 86/13**

(45) Publication of the grant of the patent:
**17.04.91 Bulletin 91/16**

(84) Designated Contracting States:
**AT BE CH DE FR GB LI LU NL SE**

(56) References cited:
**EP-A- 0 146 354**
**WO-A-83/04053**

**NUCLEIC ACIDS RESEARCH, vol. 11, no. 6, March 1983, Oxford (GB); Rik Derynck et al.:"Expression of the human interferon-gamma cDNA in yeast", pp. 1819-1837**

**BIOCHIMICA ET BIOPHYSICA ACTA, 695 (1982), Elsevier Biomedical Press, Pravin-kumar B. Sehgal: "The interferon genes", pp. 17-33**

(73) Proprietor: **Biogen, Inc.**
**Fourteen Cambridge Center**
**Cambridge, Massachusetts 02142(US)**

(72) Inventor: **ALLET, Bernard**
**Rue des Bossons, 20**
**CH-1213 Onex(CH)**

(74) Representative: **Bannerman, David Gardner et al**
**Withers & Rogers 4 Dyer's Buildings Holborn London, EC1N 2JT(GB)**

## Description

### TECHNICAL FIELD OF THE INVENTION

This invention relates to a modified gamma interferon, to DNA sequences that code for it, and to processes for making it. More particularly, the invention relates to an amino-Δ3-gamma interferon which is more soluble, and accordingly easily purifiable, and which is highly stable and retains at least a similar level of antiviral and antiproliferative biological activity as full length mature human gamma interferon. This invention also relates to the DNA sequence coding for the amino-Δ3-gamma interferon, and to processes for expressing that DNA sequence to produce the amino-Δ3-gamma interferon. Finally, the invention relates to methods and DNA sequences for improving the expression levels of DNA sequences encoding selected proteins and polypeptides, including but not limited to gamma interferon or amino-Δ3-gamma interferon.

### BACKGROUND ART

In this application we will use the interferon nomenclature announced in Nature , 286, p. 110 (July 10, 1980). "IFN" will designate interferon, "IFN-α" will designate leukocyte interferon "IFN-β" will designate fibroblast interferon, and "IFN-γ" will designate immune or gamma interferon. The amino-Δ3-gamma interferon of this invention which has three amino acids removed from its amino terminal end will be designated amino-Δ3-IFN-γ. We will also use the conventional three-letter symbols for amino acids as defined in A. Lehninger, Biochemistry , pp. 73-75 (2d ed. 1975).

IFN is a cellular protein displaying antiviral activity against a broad range of viruses through induction of cellular RNA and protein synthesis directed against virus replication. For example, human IFN has been used to combat the viral activity of the following: respiratory infections [Texas Reports on Biology and Medicine , Vol. 35, pp. 486-96 (1977) (hereinafter referred to as Texas Reports )]; herpes simplex keratitis [Texas Reports , pp. 497-500; R. Sundmacher, "Exogenous Interferon In Eye Diseases", International Virology IV , The Hague, Abstract nr. w2/11, p. 99 (1978)]; acute hemorrhagic conjunctivitis [Texas Reports , pp. 501-10]; adenovirus keratoconjunctivitis [A. Romano et al., ISM Memol-A8131 (October, 1979)]; varicella-zoster [Texas Reports , pp. 511-15]; cytomegalovirus infection [Texas Reports , pp. 523-27]; and hepatitis B [Texas Reports , pp. 516-22]. See also W. E. Stewart, II, The Interferon System, pp. 307-21, Springer-Verlag (2 ed.) (1981) (hereinafter referred to as The Interferon System ).

IFN has other effects in addition to its antiviral action. For example, it antagonizes the effect of colony stimulating factor, inhibits the growth of hemopoietic colony-forming cells and interferes with the normal differentiation of granulocyte and macrophage precursors [Texas Reports , pp. 343-49]. It also inhibits erythroid differentiation in DMSO-treated Friend leukemia cells [Texas Reports , pp. 420-28].

IFN may also play a role in the regulation of the immune response. For example, depending upon the dose and time of application in relation to antigen, IFN can be both immunopotentiating and immunosuppressive in vivo and in vitro [Texas Reports , pp. 357-69]. In addition, IFN is also known to enhance the activity of killer lymphocytes and antibody-dependent cell-mediated cytotoxicity [R. R. Herberman et al., "Augmentation By Interferon Of Human Natural And Antibody-Dependent Cell-Mediated Cytotoxicity", Nature , 227, pp. 221-23 (1979); P. Beverley and D. Knight, "Killing Comes Naturally", Nature , 278, pp. 119-20 (1979); Texas Reports , pp. 375-80; J. R. Huddlestone et al., "Induction And Kinetics Of Natural Killer Cells In Humans Following Interferon Therapy", Nature , 282, pp. 417-19 (1979); S. Einhorn et al., "Interferon And Spontaneous Cytotoxicity In Man. II. Studies In Patients Receiving Exogenous Leukocyte Interferon", Acta Med. Scand. , 204, pp. 478-83 (1978)].

Killer lymphocytes and antibody-dependent cell-mediated cytotoxicity may be directly or indirectly involved in the immunological attack on tumor cells. Therefore, in addition to its use as an antiviral agent, IFN has potential application in antitumor and anticancer therapy and in immunomodulation agents and methods [The Interferon System , pp. 319-21, 250-56]. It is now known that IFNs affect the growth of many classes of tumors in many animals [The Interferon System , pp. 292-304].

Interferons, like other antitumor agents, seem most effective when directed against small tumors. The antitumor effects of animal IFN are dependent on dosage and time, but have been demonstrated at concentrations below toxic levels. Accordingly, numerous investigations and clinical trials have been and continue to be conducted into the antitumor and anticancer properties of human IFNs. These include treatment of several malignant diseases such as osteosarcoma, acute myeloid leukemia, multiple myeloma and Hodgkin's disease [Texas Reports , pp. 429-35]. Although the results of these clinical tests are encouraging, the antitumor, anticancer and immunomodulation applications of human IFNs have been severely hampered by lack of an adequate supply of purified IFN.

Interferon therapy against viruses and tumors or cancers has been conducted at varying dosage regimes and under several modes of administration [The Interferon System , pp. 306-22]. For example, interferon has been effectively administered orally, by inoculation -- intravenous, intramuscular, intranasal, intradermal and subcutaneous -- and in the form of eye drops, ointments and sprays. It is usually administered one to three times daily in dosages of $10^4$ to $10^8$ units.

The extent of the therapy and the dosage regime depends on the patient and the condition being treated. For example, virus infections are usually treated by daily or twice daily doses over several days to two weeks and tumors and cancers are usually treated by daily or multiple daily doses over several months or years. The most effective therapy for a given patient must, of course, be determined by the attending physician, who will consider such well known factors as the course of the disease, previous therapy, and the patient's response to interferon in selecting a mode of administration and a dosage regime.

Interferons have been classified into two groups: Type I and Type II IFNs. Type I IFNs are the "classical" acid stable IFNs induced by viruses or synthetic polynucleotides and generally consist of two species: IFN-αs and IFN-β. Type II IFN consists of only one species designated as IFN-γ. IFN-γ is also referred to in the art as immune or gamma interferon.

IFN-γ is a glycoprotein induced in lymphocytes by specific antigen or various mitogens and is antigenically distinct from IFN-α and IFN-β [A. Mizrahi et al., "Glycosylation Of Interferon", J. Biol. Chem. , 253, pp. 7612-15 (1978); The Interferon System , pp. 107-08; P. Gray et al., "Expression Of Human Immune Interferon cDNA In E. coli And Monkey Cells", Nature , 295, 503-08 (1982); M. P. Langford et al., "Large-Scale Production And Physicochemical Characterization Of Human Immune Interferon", Infection And Immunity , 26, pp. 36-41 (1979)]. The protein has been reported to have a molecular weight of 40,000-46,000 daltons, with the possibility that its glycosylated form has a molecular weight of 65,000-70,000 daltons. The absence of glycosylation, however, does not substantially affect the biological activity of the protein. Moreover, laboratory tests of both rDNA glycosylated and rDNA non-glycosylated IFN-γ have indicated that the non-glycosylated IFN-γ may be slightly less toxic and slightly more active as an anticancer agent than glycosylated IFN-γ. However, non-glycosylated IFN-γ may be slightly less active as an antiviral agent.

In addition to being acid labile (at pH2), IFN-γ has been reported to be inactivated after 1 hour at 56°C. See also M. deLey et al., "Interferon Induced In Human Leukocytes By Mitogens: Production, Partial Purification And Characterization", Eur. J. Immunol. , 10, pp. 877-83 (1980); Y. K. Yip et al., "Partial Purification And Characterization Of Human γ (Immune) Interferon", Proc. Natl. Acad. Sci. USA , 78, pp. 1601-05 (1981). It has been reported that IFN-γ recognizes a different cell receptor than IFN-α or IFN-β [A. A. Branca et al., "Evidence That Types I And II Interferons Have Different Receptors", Nature , 294, pp. 768-70 (1981)]. Moreover, IFN-γ has been demonstrated in various laboratory studies to have potency against certain viruses, such as herpes genitalis.

In addition to its antiviral activity, IFN-γ displays antitumor or anticancer activity. Various studies indicate that IFN-γ works both directly and indirectly to inhibit the growth of, or to kill, certain tumor cells. Moreover, as compared to IFN-α and IFN-β, IFN-γ's antitumor activity, at least in mice, results in tumor regression. In addition, its activation of natural killer cells does not reach a plateau, as observed for IFN-α and IFN-β. IFN-γ is also less inhibited by circulating levels of gangliosides than are IFN-α and IFN-β [H. Ankel et al., "Mouse Fibroblast (Type I) And Immune (Type II) Interferons: Pronounced Differences In Affinity For Gangliosides And In Antiviral And Antigrowth Effects On Mouse Leukemia L-1210R Cells", Proc. Natl. Acad. Sci. USA , 77, pp. 2528-32 (1980)]. Therefore, cells or tumors, particularly solid tumors, e.g., lung, breast and colon-rectum cancers, that display a poor response to IFN-α or IFN-β may be effectively treated with IFN-γ [e.g., Crane et al., J. Natl. Cancer Institute , 61, p. 891 (1978); Barn et al., Abstract N.Y. Acad. Sci. , No. 11 (October 23-26, 1979); Blalock et al., Cellular Immunology , 49, pp. 390-94 (1980); B. Y. Rubin et al., "Differential Efficacies Of Human Type I And Type II Interferons As Antiviral And Antiproliferative Agents", Proc. Natl. Acad. Sci. USA , 77, pp. 5928-32 (1980)].

Another use of IFN-γ may be as an immunoregulatory agent. The antiproliferative effect of IFN-γ on transformed cells has been reported to be 10 to 100 times greater than that of IFN-α or IFN-β [P. W. Gray et al., supra ].

The DNA sequence coding for human IFN-γ has been cloned and utilized to transform various hosts which then produce IFN-γ [e.g., W. Fiers et al., "The Human Fibroblast And Human Immune Interferon Genes And Their Expression In Homologous And Heterologous Cells", Phil. Trans. R. Soc. Lond. , B 299, pp. 29-38 (1982); P. W. Gray et al., "Expression Of Human Immune Interferon cDNA in E.coli And Monkey Cells", Nature , 295, pp. 503-08 (1982)]. Various expression control sequence - IFN-γ coding sequence constructions now exist that enable IFN-γ to be produced in appropriately transformed hosts at a high level (≧ 10% of all protein). Such rDNA production of IFN-γ was expected to solve the previous acute shortage of the protein for clinical studies in antiviral, anticancer and immunomodulation methods and agents. However, it has not.

Instead, as the transformed cell accumulates large amounts of the foreign IFN-γ protein, the IFN-γ molecules interact with each other to form highly insoluble aggregates not typically found in the normal cell. The cell then may react to this accumulation of protein by forming inclusion bodies into which the foreign protein aggregates are packaged. As a result of this insolubility and cellular packaging, the IFN-γ-containing aggregates may be isolated from the cell. However, after extraction and resuspension in buffer, it has been very difficult to purify the IFN-γ from the bacterial and other cellular extracts of the variety of hosts in which it has been produced. These purification problems have, therefore, hindered efforts to make IFN-γ available in the amounts needed for use in antiviral, anticancer and immunomodulation methods and compositions.

DISCLOSURE OF THE INVENTION

The present invention solves the problems referred to above by providing a more soluble, and accordingly easily purifiable, amino-Δ3-IFN-γ in which three amino acid residues have been deleted from the amino terminal end of the IFN-γ as compared to full length IFN-γ. This deletion does not substantially change the biological activity of the protein. Yet, it provides an IFN-γ-like protein that is highly stable, more soluble, and therefore more easily purifiable, than previously made recombinant IFN-γ.

The amino-Δ3-IFN-γ of this invention may be used in antiviral, antiproliferative or immunomodulative compositions, such as those comprising a pharmaceutically effective amount of the protein, or in antiviral, antiproliferative or immunomodulative methods characterized by the step of treating a cell in a pharmaceutically acceptable manner with a pharmaceutically effective amount of such compositions.

This invention also includes the DNA sequences coding for the amino-Δ3-IFN-γ and the methods for using those sequences to produce the amino-Δ3-IFN-γ. More preferably, the DNA sequences of this invention are additionally modified by a deletion of nucleotides in the 3' non-coding region of the IFN-γ gene but maintaining at least the poly G tail at the carboxy terminal end of the non-coding region. This deletion further enhances the expression of the gene within the various transformed hosts and results in increased yields of amino-Δ3-IFN-γ. It may also be used alone to enhance the expression levels of other IFN-γ-like polypeptides. Recent publications, including EP-A-146 354 (Genentech), WO 83/04053 (Applied Molecular Genetics), Derynk et al. Nucleic Acids Research , 11(6), 1819-37 (1983) and Pravin kumar B. Sehgal, Biochimica et Biophysica Acta , 695, 17-33 (1982), have referred to the expression of heterologous genes, including IFN-γ genes, in various hosts, but the methods described herein for improving expression, e.g., by deletion in the 3' non-coding region of the heterologous gene, are not mentioned.

Furthermore, such a 3' non-coding deletion in the non-coding region of any DNA sequence coding for a selected protein or polypeptide and carrying a 3' poly G tail enhances the expression levels of that DNA sequence in the transformed host cell and improves the levels of selected protein production in that cell.

BRIEF DESCRIPTION OF THE DRAWINGS

Figure 1 is a schematic outline of one embodiment of a process of this invention for preparing a recombinant DNA molecule containing a nucleotide deletion in the non-coding region of the cloned IFN-γ gene.

Figure 2 is a schematic outline of one embodiment of a process of this invention for preparing a recombinant DNA molecule containing the nucleotide deletion referred to above and the DNA sequence coding for amino-Δ3-IFN-γ.

Figure 3 is a schematic depiction of relevant portions of plasmid per ori γ.

Figure 4 is a depiction of a synthetic nucleotide sequence and its use in producing the nine nucleotide deletion in the coding sequence of IFN-γ to produce a DNA sequence encoding amino-Δ3-IFN-γ.

Figure 5 is a depiction of the DNA sequence of the IFN-γ-containing insert of a recombinant DNA molecule useful as a starting material in the methods of this invention.

BEST MODE OF CARRYING OUT THE INVENTION

In order that the invention herein described may be more fully understood, the following detailed description is set forth.

In the description, the following terms are employed:

DNA Sequence --
A linear array of nucleotides connected one to the other by phosphodiester bonds between the 3' and 5' carbons of adjacent pentoses.

Polypeptide --

A linear array of amino acids connected one to the other by peptide bonds between the $\alpha$-amino and carboxy groups of adjacent amino acids.

IFN-$\gamma$-Like Polypeptide --

A polypeptide displaying substantially the same biological and immunological activity of mature IFN-$\gamma$.

Expression --

A combination of transcription and translation by which a DNA sequence encoding a particular polypeptide produces the polypeptide in a host containing the DNA sequence.

Expression Control Sequence --

A sequence of nucleotides that controls and regulates expression of DNA sequences when operatively linked to those sequences. Expression control sequences include, for example, the lac system, the trp system, the major operator and promoter regions of phage $\lambda$, the control region of fd coat protein, the TAC and TRC systems, and other sequences known to control the expression of DNA sequences of prokaryotic or eukaryotic cells and their viruses or combinations of those sequences.

Cloning Vehicle --

A plasmid, phage DNA or other DNA sequence which is able to replicate in a host cell, characterized by one or a small number of endonuclease recognition sites at which such DNA sequences may be cut in a determinable fashion without attendant loss of an essential biological function of the DNA, e.g., replication, production of coat proteins or loss of promoter or binding sites, and which contains a marker suitable for use in the identification of transformed cells, e.g., tetracycline resistance or ampicillin resistance. A cloning vehicle is also called a vector.

Cloning --

A process for obtaining a population of organisms or DNA sequences derived from one such organism or sequence by asexual reproduction.

Recombinant DNA Molecule or Hybrid DNA --

A molecule consisting of segments of DNA from different genomes which have been joined end to end.

This invention provides a stable, biologically active amino-$\Delta$3-IFN-$\gamma$, which lacks at its amino terminal end the three amino acid sequence of cysteine-tyrosine-cysteine of mature IFN-$\gamma$. This modified IFN-$\gamma$, referred to as amino-$\Delta$3-IFN-$\gamma$, also displays increased solubility as compared to full length recombinant IFN-$\gamma$. It is, therefore, more easily purifiable from cellular extracts than full length recombinant IFN-$\gamma$.

While not wishing to be bound by theory, applicants believe that the improved solubility of their amino-$\Delta$3-IFN-$\gamma$ is due to the elimination of the cysteine residues at the amino terminal end. Cysteines have sulfhydryl side chains which tend to form intermolecular disulfide linkages between individual IFN-$\gamma$ molecules. Accordingly, these linkages result in highly insoluble protein aggregates within the cell. Such insoluble aggregates complicate purification procedures. Thus, the elimination of the cysteine residues from the amino terminal end of IFN-$\gamma$ prevents these sulfhydryl interactions and provides a more soluble and easily purifiable amino-$\Delta$3-IFN-$\gamma$.

Production of the amino-$\Delta$3-IFN-$\gamma$ of this invention begins with the preparation of a DNA sequence coding for that modified interferon. This DNA sequence has a deletion of nine nucleotides at the amino terminal end of the DNA sequence encoding mature IFN-$\gamma$. Accordingly, on expression, a protein is produced having the first three amino acids, cysteine-tyrosine-cysteine, deleted from mature IFN-$\gamma$. More preferably, an additional number of nucleotides of the 3' non-coding (non-translated) region of the IFN-$\gamma$ gene are also deleted in the DNA sequences of this invention. This latter, and more preferred, construction results in a higher degree of expression of the amino-$\Delta$3-IFN-$\gamma$ coding sequence in host cells transformed with these sequences. In addition, such a deletion in the 3' non-coding region of any DNA sequence coding for a selected protein or polypeptide and carrying a 3' poly G tail results in a higher degree of expression of that protein or polypeptide in transformed host cells. Accordingly, the use of such deletions are part of this invention.

Any of a large number of expression vectors, expression control sequences and hosts may be used in combination with the DNA sequences of this invention to produce the desired amino-$\Delta$3-IFN-$\gamma$. For example, useful expression vectors may consist of segments of chromosomal, non-chromosomal and synthetic DNA sequences, such as various known derivatives of SV40 and known bacterial plasmids, e.g., plasmids from E.coli including col EI, pCRI, pBR322, pMB9 and their derivatives, wider host range plasmids, e.g., RP4, phage DNAs, e.g., the numerous derivatives of phage $\lambda$, e.g., NM 989, and other DNA phages, e.g., M13 and Filamentous single-stranded DNA phages and vectors derived from combinations of plasmids and phage DNAs such as plasmids which have been modified to employ phage DNA or other expression control sequences or yeast plasmids such as the 2$\mu$ plasmid or derivatives thereof. Useful cloning or expression hosts may include bacterial hosts such as E.coli HB 101, E.coli X1776, E.coli X2282, E.coli MRCI and strains of Pseudomonas , Bacillus subtilis , Bacillus stearothermophilus and other bacilli, yeast and other fungi, animal or plant hosts such as animal (including human) or plant cells in culture or other hosts. It should be understood that the selection of a particular host will determine whether or not the amino-Δ3-IFN-γ is glycosylated or non-glycosylated. For example, if made in mammalian cells, e.g., CHO cells, the amino-Δ3-IFN-γ will be glycosylated, while if made in E.coli, it will be non-glycosylated. Both forms of amino-Δ3-IFN-γ are included within this invention.

Various expression control sequences may be employed. These include, for example, the operator, promoter and ribosome binding and interaction sequences (including sequences such as the Shine-Dalgarno sequences) of the lactose operon of E.coli ("the lac system"), the corresponding sequences of the tryptophan synthetase system of E.coli ("the trp system"), the major operator and promoter regions of phage λ ($O_LP_L$ and $O_RP_R$), a control region of Filamentous single-stranded DNA phages, or other sequences which control the expression of genes of prokaryotic or eukaryotic cells and their viruses or combinations thereof.

Of course, not all vector-expression control sequence-host combinations may be equally efficient. The particular selection of a specific combination may be made by those of skill in the art after due consideration of the principles set forth without departing from the scope of this invention.

It should also be understood that because neither the coding sequence for mature IFN-γ nor the coding sequence for amino-Δ3-IFN-γ begin with an ATG start codon, this translational start signal must be inserted in front of the coding sequence before expression. Such constructions of an ATG-coding sequence combination may be done by those of skill in the art without departing from the scope of this invention. These constructions enable the production in appropriate transformed hosts of f-met-IFN-γ or f-met-amino-Δ3-IFN-γ. However, heterogeneity due to the presence or absence of the amino terminal Met cannot be excluded because some hosts, e.g., E.coli , are known to cleave to some extent amino terminal methionines.

In order that this invention and the methods of using it may be more fully understood, the following example is provided. This example is for illustrative purposes only. Other means and methods may be employed to construct, to produce and to use the amino-Δ3-IFN-γ of this invention without departing from the scope hereof.

## EXAMPLE

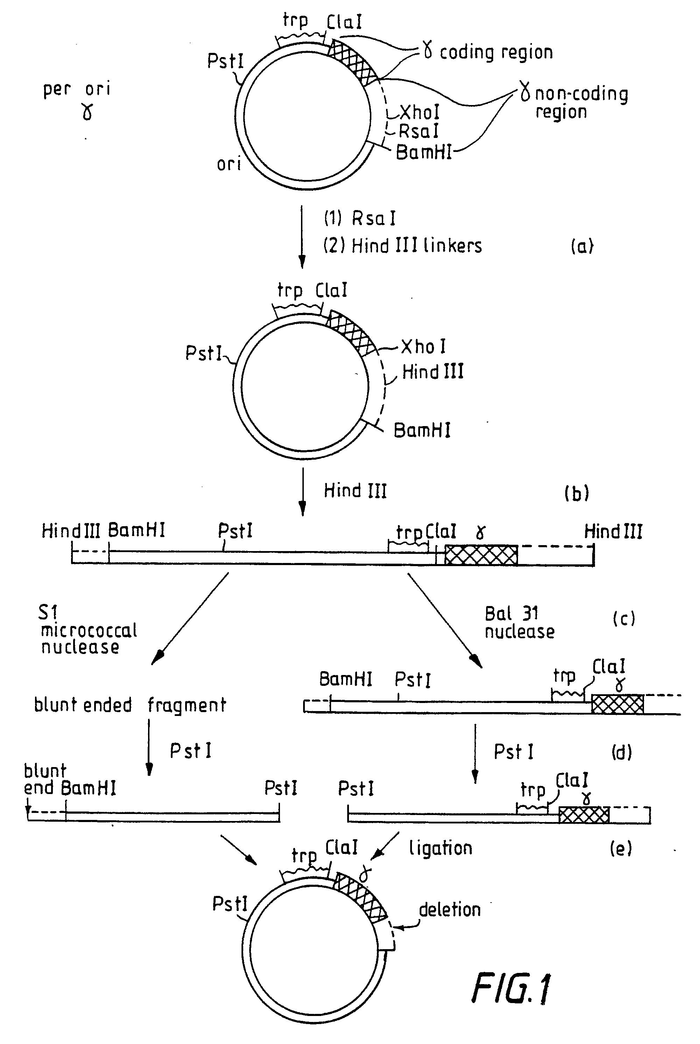

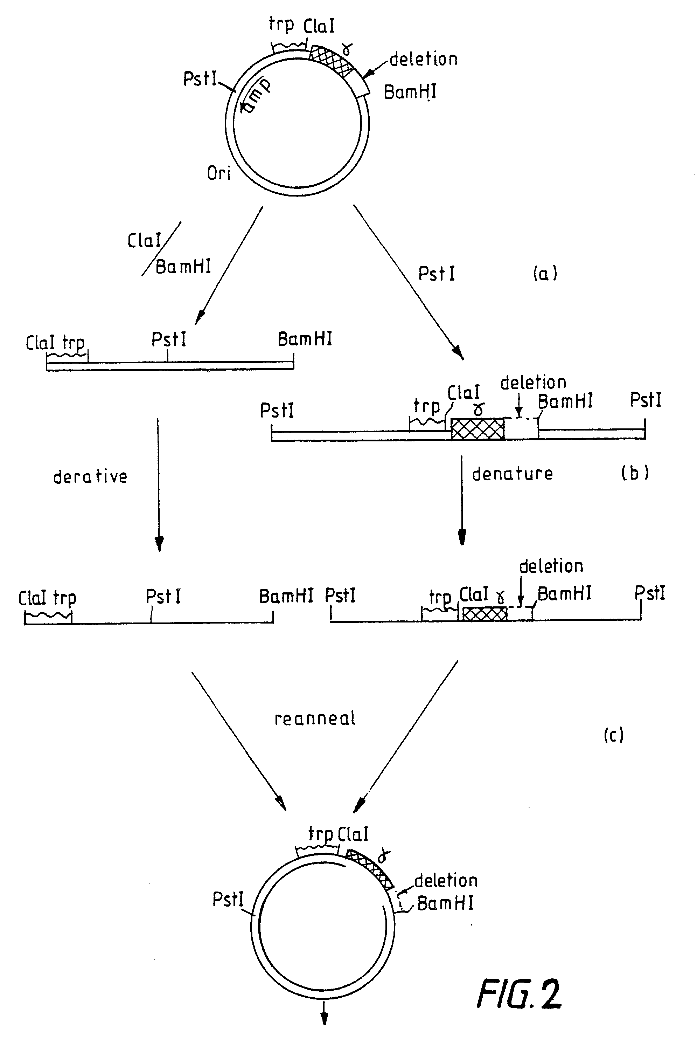

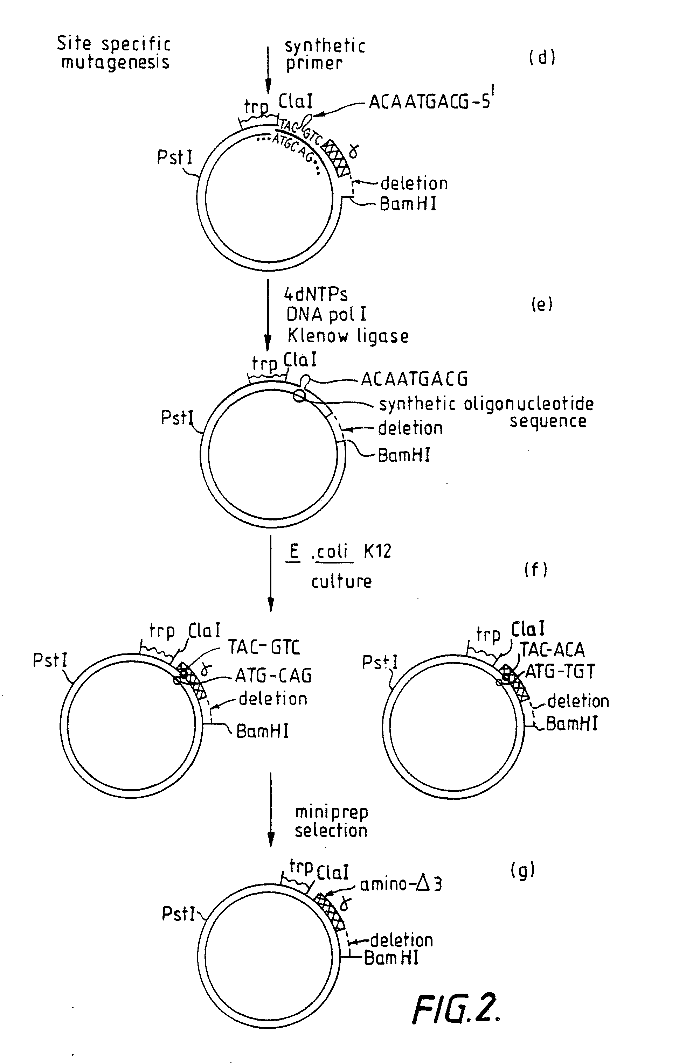

Referring to Figures 1 and 2, we have shown therein a schematic outline of one embodiment of a process for preparing the more preferred DNA sequence of this invention. This sequence encodes

amino-Δ3-IFN-γ and also carries a 422-nucleotide deletion in the 3' non-coding region of IFN-γ. According to this embodiment, we first deleted 422 nucleotides from the 3' non-coding region of the IFN-γ gene by nuclease digestion. We then deleted the nine nucleotides after the ATG start codon in the 5' coding region (amino terminal end) of the IFN-γ gene by site specific mutagenesis.

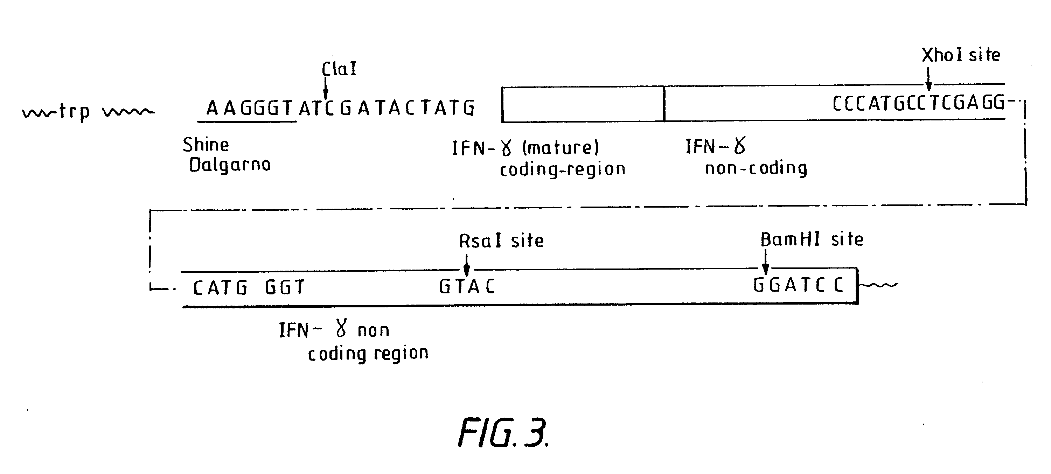

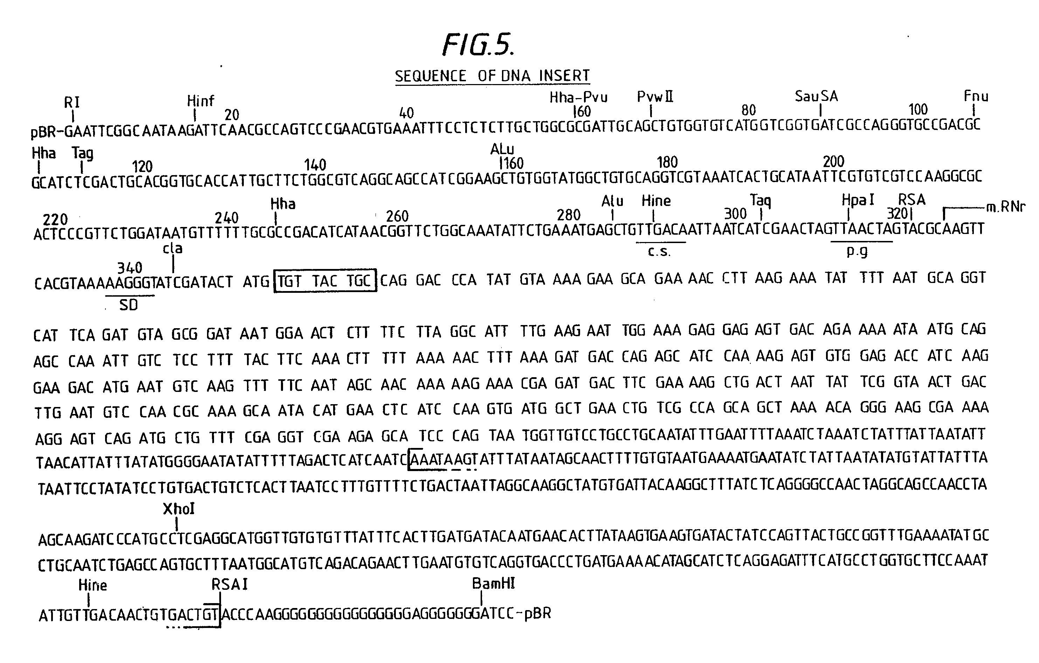

The starting material used in the embodiment of this invention described here is recombinant DNA molecule per ori γ (Figure 1(a)) which contains the DNA sequence encoding mature IFN-γ, operatively linked to a trp -derived expression control sequence (see, e.g., Figures 3 and 5). This molecule also has a translation start signal (ATG) attached to the coding sequence (TGT) of the first amino acid of mature IFN-γ and contains a Cla I restriction site between the Shine-Dalgarno sequence of the trp expression control sequence and the start codon (Figures 3 and 5). As shown more particularly in Figures 3 and 5, this molecule also contains a portion of the 3' non-coding (non-translated) region of the IFN-γ gene with a Bam HI restriction site marking the carboxyl end of this non-coding region. The non-coding region also contains Xho I and Rsa I restriction sites within its sequence. The remainder of the molecule (from the Bam HI site to the trp sequence) is comprised of the sequences of plasmid pBR322 and contains a Pst I restriction site. Although we used this recombinant DNA molecule as our starting material in this illustrative example of our invention, it should be understood that any other source of the IFN-γ coding or non-coding region could have been equally well employed to produce our amino-Δ3-IFN-γ.

As depicted in Step (a) of Figure 1, we first restricted per ori γ with Rsa I and inserted Hind III linkers to construct a Hind III restriction site in the 3' non-coding region of the IFN-γ gene. We then restricted the resulting plasmid with Hind III and separated the linearized DNA into two aliquots (Step (b), Figure 1).

As depicted in Step (c) of Figure 1, we treated one aliquot of the above Hind III cleaved DNA with SI micrococcal nuclease to blunt end the residues of the Hind III restriction cut. We purified this DNA on an agarose gel, restricted it with Pst I, and purified the larger resulting fragment (containing the Bam HI site) on an agarose gel (Step (d), Figure 1).

We digested the other aliquot of the Hind III cleaved DNA with Bal 31 nuclease, an enzyme that digests double-stranded DNA, under conditions that removed 422 nucleotides from each end of the linear DNA (Step (c), Figure 1). After purification of the digested DNA on an agarose gel, we restricted the DNA with Pst I and purified the smaller resulting fragment (containing the trp control sequence, the coding region of the IFN-γ gene and a portion of the beginning of the non-coding region of the IFN-γ gene) (Step (d), Figure 1). The non-coding region of the IFN-γ gene on the selected fragment had been digested from the Hind III site toward the coding region of the IFN-γ gene, resulting in a 422-nucleotide deletion in that region. This deletion is depicted by the brackets in Figure 5.

The two DNA fragments produced above were then ligated to form a recircularized DNA characterized by a 422-nucleotide deletion in the 3' non-coding region of the IFN-γ gene (Step (e), Figure 1). This deletion results in a higher level of expression of the IFN-γ gene in cells transformed with the molecule, i.e., approximately 20-30% of the total cellular protein. This deletion is useful in the expression of any IFN-γ coding sequence. Accordingly, it need not be combined as below with the coding sequence for amino-Δ3-IFN-γ. Instead, it may be advantageously employed in the production of other IFN-γs and IFN-γ-like polypeptides.

By use of Xba I linkers and allowing longer Bal 3I nuclease digestion as described above, we also constructed a DNA characterized by a 484-nucleotide deletion in the 3' non-coding region of the IFN-γ gene. This deletion provides an equal or somewhat greater yield of IFN-γ as is obtained with the 422-nucleotide deletion. Utilizing the methods of the invention, therefore, a deletion of any nucleotide length in the 3' non-coding region of the IFN-γ gene may be constructed to provide increased yields of IFN-γ.

After incorporation of the above-described deletion into the IFN-γ gene, we then introduced a 9-nucleotide deletion in the 5' coding region of the IFN-γ gene to produce a coding sequence for amino-Δ3-IFN-γ. It should be understood that while we first manipulated our starting recombinant DNA molecule, per ori γ, to introduce the 422-nucleotide non-coding deletion, we could as well have introduced the 9-nucleotide deletion in the amino terminal end first or even not combined that deletion with the 422-nucleotide non-coding deletion. However, preferably, our expression vehicle carries both deletions because high levels of expression are obtained.

As shown in Figure 2, we treated the recircularized molecule in two ways. We restricted one aliquot of the DNA with Cla I and Bam HI, using the conditions recommended by the suppliers of those endonuclease restriction enzymes, and isolated and purified the larger Cla I-Bam HI fragment on an agarose gel (Step (a), Figure 2). The fragment contains a part of the trp expression control sequence, a Pst I restriction site and a part of the 3' non-coding region of the IFN-γ gene (Figure 2b). We then denatured this fragment (which is double-stranded) in 0.1N NaOH at room temperature for 10 min to produce single-stranded Cla I-Bam HI DNA fragments (Step (b), Figure 2).

We restricted the second aliquot of our recircularized molecule with Pst I to linearize it and purified the linear DNA on an agarose gel (Step (a), Figure 2). We then denatured this linear double-stranded DNA in 0. 1N NaOH at room temperature for 10 min to produce single-stranded DNAs (Step (b), Figure 2).

We next mixed the single-stranded DNA fragments produced by the two above-described restrictions at 60°C in Tris-HCl(pH 8)-EDTA-NaCl ("TEN") hybridization buffer for 2-3 h (Step (c), Figure 2). This reannealed the DNA fragments to form a renatured double-stranded plasmid with a gap in one strand between the Cla I and Bam HI restriction sites (Figure 2). We then purified the renatured DNA by precipitation in ethanol and redissolved the purified DNA in ligase/polymerase buffer.

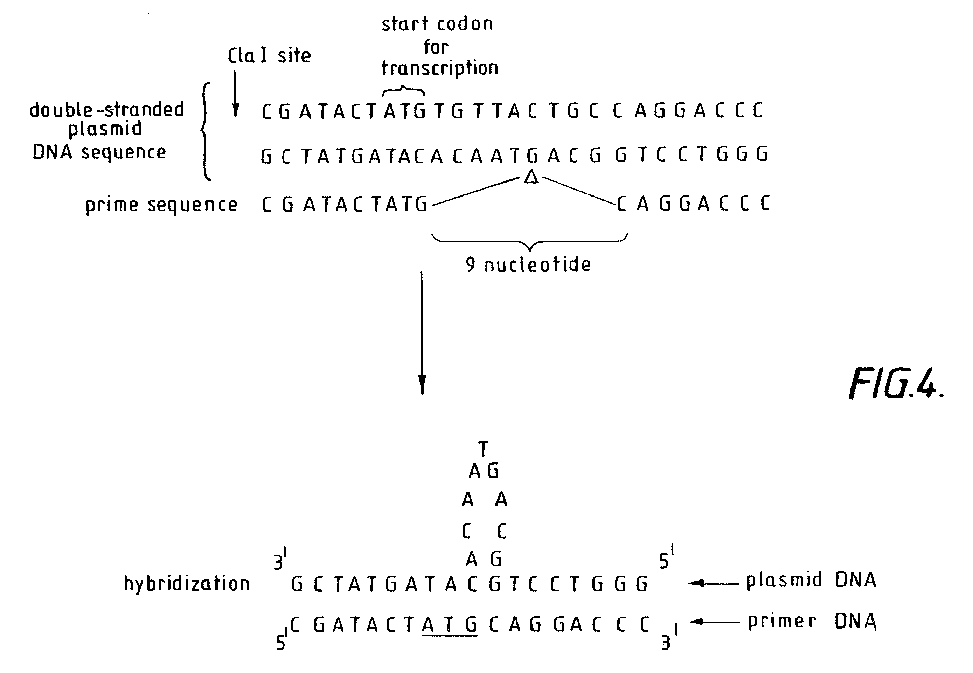

We next prepared a synthetic oligonucleotide primer, having the sequence CGATACTATGCAGGACCC. As depicted in Figure 4, this primer is complementary to the DNA sequence of molecule per ori γ in the region of the trp control sequence and the coding region of the IFN-γ gene. However, the primer carries a deletion of nine nucleotides as compared to that DNA sequence of molecule per ori γ. The deleted sequence, TGTTACTGC, corresponds to the coding sequence of the first three amino terminal amino acids cysteine-tyrosine-cysteine of IFN-γ. Accordingly, the primer after hybridization to one strand of per ori γ enables the synthesis of a DNA sequence lacking those 9-nucleotides.

We hybridized the above-described primer to the single-stranded DNA portion of our renatured DNA, described above (Step (d), Figure 2). We affected this hybridization in ligase/polymerase buffer at 17°C to ensure that the 9-nucleotide deletion would not prevent hybridization. We then filled in the single-stranded DNA gap using the four deoxyribonucleotide triphosphates, DNA polymerase I (Klenow fragment) and ligase (Step (e), Figure 2). This procedure results in the production of a double-stranded DNA having on one strand the complete coding sequence of IFN-γ and having on the other strand a DNA sequence lacking the nine nucleotides which code for the first three amino terminal amino acids of IFN-γ (Figure 4).

We then transformed E.coli K12 cells with our filled-in, double-stranded DNA and selected those hosts that had been transformed with the DNA sequence carrying the nine nucleotide deletion on one strand (Step (f), Figure 2). For selection, we used our synthetic primer sequence as a probe in a Grunstein-Hogness hybridization screening at 42°C [H. Grunstein and D. S. Hogness, "Colony Hybridization: A Method For The Isolation Of Cloned DNAs That Contain A Specific Gene", Proc. Natl. Acad. Sci. USA, 72, pp. 3961-65 (1975)]. During hybridization selection we distinguished between weak signals (where a mismatch occurred) and strong signals (where there was no mismatch).

We took the selected hosts (strong signal, no mismatch with probe) that contained a DNA sequence carrying on one strand the desired 9-nucleotide deletion, grew them up in rich medium, lyzed them, and observed two protein bands, one corresponding to the full length IFN-γ and the other corresponding to the amino-Δ3-IFN-γ. The reason we observed two bands is that the cultured cells, having undergone replication, now contain two types of molecules. (Step (f), Figure 2). One is formed by replication of the DNA strand that contains the full length IFN-γ coding sequence and the other is formed by replication of the DNA strand that has the 9-nucleotide deletion following the start codon of the IFN-γ gene. Accordingly, we made minipreps from the positive host cells and separated the two types of plasmids using Grunstein-Hogness hybridization selection with the synthetic probe at 42°C as before, selecting the strongly-positive plasmids (Step (g), Figure 2).

We then transformed E.coli K12 cells with these selected plasmids, now containing only DNA sequences having the nine nucleotide deletion at the 5' end of the IFN-γ coding sequence (see the nucleotide sequence within the box in Figure 5). After culturing these hosts, we observed a protein band for the Amino-Δ3-IFN-γ. No band was observed for full length IFN-γ. We have designated this plasmid carrying DNA sequences with a nine nucleotide deletion at the 5' end of the IFN-γ gene and a 422-nucleotide deletion in the 3' non-coding region: ptrp-amino-Δ3-IFN-γ-Δ422. We have designated the host transformed with this plasmid which produces the amino Δ3-IFN-γ: E.coli K12 (ptrp-amino-Δ3-IFN-γ-Δ422).

The amino-Δ3-IFN-γ prepared above was then compared with full length IFN-γ, prepared from E.coli hosts transformed with per ori γ, in terms of its antiviral and antiproliferative activities. This comparison demonstrated that the amino-Δ3-IFN-γ had at least similar antiviral and antiproliferative activities to recombinant IFN-γ from per ori γ. Moreover, the amino-Δ3-IFN-γ was highly soluble and accordingly easy to purify. It was also very stable in solution.

It should, of course, be understood that while we have described one method for producing amino-Δ3-IFN-γ, a variety of other methods may also be employed to prepare this modified IFN-γ. For example, at the DNA level, other methods may be used to produce the coding sequence of amino-

Δ3-IFN-γ. These include synthetic methods and other mutagenesis and deletion methods. At the protein level, a variety of techniques also exist to produce amino-Δ3-IFN-γ.

It should also be understood that while the above example is directed to an amino-Δ3-IFN-γ encoded by a DNA sequence also having an additional 422- or 484-nucleotide deletion in the non-coding region of the IFN-γ gene, this invention is not so limited. Rather, it includes DNA sequences containing a nucleotide deletion in the 3' non-coding region which may be of varying length, the deletion preferably starting at the Rsal endonuclease recognition site and continuing toward the coding region of the IFN-γ gene. In addition, the invention includes DNA sequences coding for amino-Δ3-IFN-γ which contain only the nine nucleotide deletion at the amino terminal end of the coding region of the gene. In such an instance, only the schematic outline of Figure 2, for example, would be used and Xho I restriction instead of Bam HI restriction would be preferred.

Furthermore, the present invention also extends to DNA sequences coding for any protein or polypeptide and carrying a 3' poly G tail, the DNA sequences containing a nucleotide deletion in the 3' non-coding region of the gene but maintaining at least the poly G tail at the carboxy terminal end of the non-coding region. This deletion enhances the level of expression of the DNA sequences in host cells transformed with those DNA sequences and results in increased yields of the protein or polypeptide.

Microorganisms, recombinant DNA molecules, and DNA sequences prepared by the processes of this invention are exemplified by cultures deposited in the culture collection Deutsche Sammlung von Mikroorganismen in Gottingen, West Germany on March 12, 1984:

E.coli K12 (ptrp-amino-Δ3-IFN-γ)
E.coli K12 (ptrp-amino-Δ3-IFN-γ-Δ422)
E.coli K12 (ptrp-amino-Δ3-IFN-γ-Δ484)

These cultures were assigned accession numbers DSM 2920, 2921 and 2922, respectively.

While we have herein presented a number of embodiments of this invention, it is apparent that our basic construction can be altered to provide other embodiments which utilize the process and composition of this invention. Therefore, it will be appreciated that the scope of this invention is to be defined by the claims appended hereto rather than by the specific embodiments which have been presented hereinbefore by way of example.

## Claims

1. A method for improving the level of expression of a DNA sequence coding for a selected protein or polypeptide and carrying a 3' poly G tail in a host cell transformed with a recombinant DNA molecule containing the DNA sequence, comprising the steps of deleting at least a portion of the 3' non-coding region of the DNA sequence coding on expression for the protein or polypeptide but maintaining the poly G tail, culturing the transformed host and collecting said protein or polypeptide.

2. A DNA sequence that codes on expression for a protein or polypeptide and which includes the 3' non-coding region of the gene for said protein or polypeptide and a 3' poly G tail, said DNA sequence having a nucleotide deletion of at least a portion of the 3' non-coding region but maintaining the poly G tail at the carboxy terminal end of the non-coding region which results in enhanced expression of said DNA sequence.

3. A recombinant DNA molecule characterized by the DNA sequence of claim 2, said DNA sequence being operatively linked to an expression control sequence in said recombinant DNA molecule.

4. A host cell transformed with at least one recombinant DNA molecule according to claim 3.

5. A DNA sequence according to claim 2 that codes on expression for amino-△3-IFN-γ.

6. The DNA sequence of claim 5, wherein an ATG start codon is attached directly to the 5' end.

7. A DNA sequence according to claim 2 that on expression codes for an IFN-γ-like polypeptide and which includes the 3' non-coding region of the IFN-γ gene, and a 3' poly G tail, said DNA sequence having a nucleotide deletion of at least a portion of the 3' non-coding region but maintaining the poly G tail at the carboxy terminal end of the non-coding region of the IFN-γ gene.

8. The DNA sequence of claim 7, wherein said DNA sequence has a deletion of 422 nucleotides in the 3' non-coding region of the IFN-γ gene, said deletion starting at the Rsa I endonuclease recognition site and continuing toward the coding region of the IFN-γ gene.

9. The DNA sequence of claim 7, wherein said DNA sequence has a deletion of 484 nucleotides in the 3' non-coding region of the IFN-γ gene, said deletion starting at the Rsa I endonuclease recognition site and continuing toward the coding region of the IFN-γ gene.

10. A DNA sequence according to claim 2 that codes on expression for IFN-γ.

11. A DNA sequence according to claim 10, wherein an ATG start codon is attached directly to the 5' end.

12. The DNA sequence of claim 10, wherein said DNA sequence has a deletion of 422 nucleotides in the 3' non-coding region of the IFN-γ gene, said deletion starting at the Rsa I endonuclease recognition site and continuing toward the coding region of the IFN-γ gene.

13. The DNA sequence of claim 10, wherein said DNA sequence has a deletion of 484 nucleotides in the 3' non-coding region of the IFN-γ gene, said deletion starting at the Rsa I endonuclease recognition site and continuing toward the coding region of the IFN-γ gene.

14. A recombinant DNA molecule characterized by a DNA sequence selected from the group consisting of the DNA sequences according to any one of claims 5-13, said DNA sequence being operatively linked to an expression control sequence in said recombinant DNA molecule.

15. A host cell transformed with at least one recombinant DNA molecule according to claim 14.

Claims for the following Contracting State: AT

1. A method for improving the level of expression of a DNA sequence coding for a selected protein or polypeptide and carrying a 3' poly G tail in a host cell transformed with a recombinant DNA molecule containing the DNA sequence, comprising the steps of deleting at least a portion of the 3' non-coding region of the DNA sequence coding on expression for the protein or polypeptide but maintaining the poly G tail, culturing the transformed host and collecting said protein or polypeptide.

2. A method which comprises preparing a DNA sequence that codes on expression for a protein or polypeptide and which includes the 3' non-coding region of the gene for said protein or polypeptide and a 3' poly G tail, said DNA sequence having a nucleotide deletion of at least a portion of the 3' non-coding region but maintaining the poly G tail at the carboxy terminal end of the non-coding region which results in enhanced expression of said DNA sequence.

3. A method which comprises preparing a recombinant DNA molecule comprising a DNA sequence that codes on expression for a protein or polypeptide and which includes the 3' non-coding region of the gene for said protein or polypeptide and a 3' poly G tail, said DNA sequence having a nucleotide deletion of at least a portion of the 3' non-coding region but maintaining the poly G tail at the carboxy terminal end of the non-coding region which results in enhanced expression of said DNA sequence, said DNA sequence further being operatively linked to an expression control sequence in said recombinant DNA molecule.

4. A host cell transformed with at least one recombinant DNA molecule comprising a DNA sequence that codes on expression for a protein or polypeptide and which includes the 3' non-coding region of the gene for said protein or polypeptide and a 3' poly G tail, said DNA sequence having a nucleotide deletion of at least a portion of the 3' non-coding region but maintaining the poly G tail at the carboxy terminal end of the non-coding region which results in enhanced expression of said DNA sequence, said DNA sequence further being operatively linked to an expression control sequence in said recombinant DNA molecule.

5. A method according to claim 2, wherein said DNA sequence codes on expression for amino-Δ3-IFN-γ.

6. A method according to claim 5, wherein an ATG start codon is attached directly to the 5' end of said DNA sequence.

7. A method according to claim 2, wherein said DNA sequence codes on expression for an IFN-γ-like polypeptide and includes the 3' non-coding region of the IFN-γ gene and a 3' poly G tail, said DNA sequence having a nucleotide deletion of at least a portion of the 3' non-coding region but maintaining the poly G tail at the carboxy terminal end of the non-coding region of the IFN-γ gene.

8. A method according to claim 7, wherein said DNA sequence has a deletion of 422 nucleotides in the 3' non-coding region of the IFN-γ gene, said deletion starting at the Rsa I endonuclease recognition site and continuing toward the coding region of the IFN-γ gene.

9. A method according to claim 7, wherein said DNA sequence has a deletion of 484 nucleotides in the 3' non-coding region of the IFN-γ gene, said deletion starting at the Rsa I endonuclease recognition site and continuing toward the coding region of the IFN-γ gene.

10. A method according to claim 2, wherein said DNA sequence codes on expression for IFN-γ.

11. A method according to claim 10, wherein an ATG start codon is attached directly to the 5' end of said DNA sequence.

12. A method according to claim 10, wherein said DNA sequence has a deletion of 422 nucleotides in the 3' non-coding region of the IFN-γ gene, said deletion starting at the Rsa I endonuclease recognition site and continuing toward the coding region of the IFN-γ gene.

13. A method according to claim 10, wherein said DNA sequence has a deletion of 484 nucleotides in the 3' non-coding region of the IFN-γ gene, said deletion starting at the Rsa I endonuclease recognition site and continuing toward the coding region of the IFN-γ gene.

14. A method according to any one of claims 5-13, wherein said DNA sequence is operatively linked to an expression control sequence.

15. A host cell transformed with at least one recombinant DNA molecule comprising a DNA sequence prepared according to a method of any one of claims 5-13.

**Revendications**

1. Procédé pour améliorer le niveau d'expression d'une séquence d'ADN qui code pour une protéine ou un polypeptide choisi et qui porte une queue de 3'-poly G dans une cellule hôte transformée par une molécule d'ADN recombinant contenant la séquence d'ADN, caractérisé en ce qu'il comprend les étapes consistant à la suppression d'au moins une partie de la région qui ne code pas en 3' de la séquence d'ADN qui code pour l'expression pour la protéine ou le polypeptide qui maintient la queue de poly G, en la culture de l'hôte transformé et à recueillir la protéine ou le polypeptide.

2. Séquence d'ADN caractérisée en ce qu'elle code pour l'expression pour une protéine ou un polypeptide et qu'elle comporte la région qui ne code pas en 3' du gène pour la protéine ou le polypeptide et une queue de poly G en 3', la séquence d'ADN comportant la suppression de nucléotides d'au moins une portion de la région qui ne code pas en 3', mais qui maintient la queue de poly G à l'extrémité terminale du carboxylique de la région qui ne code pas, qui entraîne une augmentation de l'expression de la séquence d'ADN.

3. Molécule d'ADN recombinant selon la revendication 2, caractérisée en ce que la séquence d'ADN est reliée opérativement à une séquence de contrôle de l'expression dans la molécule d'ADN recombinant

4. Cellule hôte caractérisée en ce qu'elle est transformée par au moins une molécule d'ADN recombinant selon la revendication 3.

5. Séquence d'ADN selon la revendication 2, caractérisée en ce qu'elle code pour l'expression de l'amino Δ 3-Interféron gamma-(IFN-γ.)

6. Séquence d'ADN de la revendication 5, caractérisée en ce que dans celle-ci un codon de démarrage ATG est attaché directement à l'extrémité en 5'.

7. Séquence selon la revendication 2, caractérisée en ce qu'elle code pour l'expression pour un polypeptide du type IFN-γ et qu'elle comprend la région ne codant pas en 3' du gène de l'IFN-γ et une queue de poly G en 3', la séquence d'ADN comportant une suppression de nucléotides d'au moins une portion de la région ne codant en 3', mais maintenant la queue de poly G à l'extrémité terminale carboxylique de la région qui ne code pas du gène de l'IFN-γ.

8. Séquence d'ADN selon la revendication 7, caractérisée en ce qu'elle comprend la suppression de 422 nucléotides dans la région ne codant pas en 3' du gène de l'IFN-γ, la suppression partant du site de reconnaissance de l'endonuclease RsaI et se poursuivant vers la région qui code du gène de l'IFN-

9. Séquence d'ADN de la revendication 7, caractérisée en ce qu'elle comporte la suppression de 484 nucléotides dans la région ne codant pas en 3' du gène de l'IFN-γ, la suppression partant du site de reconnaissance de l'endonuclease RsaI et se poursuivant vers la région qui code du gène de l'INF-γ.

10. Séquence d'ADN selon la revendication 2, caractérisée en ce qu'elle code pour l'expression pour IFN-γ.

11. Séquence selon la revendication 10, caractérisée en ce que dans celle-ci un codon de départ ATG est attaché directement à l'extrémité en 5'.

12. Séquence d'ADN selon la revendication 10, caractérisée en ce qu'elle comporte la suppression de 422 nucléotides dans la région ne codant pas en 3' du gène de l'IFN-γ, la suppression partant du site de reconnaissance de l'endonuclease RsaI et se poursuivant vers la région qui code du gène de l'IFN

13. Séquence d'ADN selon la revendication 10, caractérisée en ce qu'elle comporte la suppression de 484 nucléotides dans la région ne codant pas en 3' du gène de l'IFN- γ la suppression partant du site de reconnaissance de l'endonuclease RsaI et se poursuivant vers la région qui code du gène de l'INF-γ.

14. Molécule d'ADN recombinant, caractérisée en ce qu'une séquence d'ADN choisie dans le groupe comportant les séquences d'ADN selon l'une des revendications 5 à 13, est reliée opérativement à une séquence de contrôle de l'expression dans la molécule d'ADN recombinant.

15. Cellule hôte caractérisée en ce qu'elle est transformée par au moins une molécule d'ADN recombinant conformément à la revendication 14.

Revendications pour les Etats contractants suivants: AT

1. Procédé pour améliorer le niveau d'expression d'une séquence d'ADN qui code pour une protéine ou un polypeptide choisi et qui porte une queue de 3'-poly G dans une cellule hôte transformée par une molécule d'ADN recombinant contenant la séquence d'ADN, caractérisé en ce qu'il comprend les étapes consistant en la suppression d'au moins une portion de la région qui ne code pas en 3' de la séquence d'ADN qui code pour l'expression pour la protéine ou le polypeptide mais qui maintient la queue de poly G, en la culture de l'hôte transformé et à recueillir la protéine ou le polypeptide.

2. Procédé caractérisé en ce qu'il comprend la préparation d'une séquence d'ADN qui code pour l'expression pour une protéine ou un polypeptide et qui comprend la région qui ne code pas en 3' du gène pour la protéine ou le polypeptide et une queue en 3' en poly G, la séquence d'ADN ayant une suppression de nucléotides d'au moins une portion de la région qui ne code pas en 3' mais maintenant la queue de poly G à l'extrémité terminale du carboxyle de la région ne codant pas, qui résulte en une augmentation de l'expression de la séquence d'ADN.

3. Procédé caractérisé en ce qu'il comprend, le fait de préparer une molécule d'ADN recombinant comportant une séquence d'ADN qui code pour l'expression pour une protéine ou un polypeptide et qui comprend la région ne codant pas en 3' du gèhne pour la protéine ou le polypeptide, et une queue en 3' de ply G, la séquence d'ADN ayant une suppression de nucléotide d'au moins une portion de la région qui ne code pas en 3' mais maintenant la queue de poly G à la portion terminale du carboxyle de la région qui ne code pas, qui résulte en une expression augmentée de la séquence d'ADN, la séquence d'ADN étant en outre reliée opérativement à une séquence de contrôle de l'expression dans la molécule d'ADN recombinant.

4. Cellule hôte transformée par au moins une molécule d'ADN recombinant caractérisée en ce qu'elle comprend une séquence d'ADN qui code pour l'expression pour une protéine ou un polypeptide et qui comporte la région qui ne code pas en 3' du gène pour la protéine ou le polyupeptide, et une queue de poly G en 3', la séquence d'ADN ayant une suppresion de nucléotide d'au moins une portion de la région ne codant pas en 3', mais maintenant la queue de poly G à l'extrémité terminale carboxylique de la région ne codant pas, qui résulte dans une augmentation de l'expression de la séquence d'ADN, la séquence d'ADN étant en outre reliée opérativement à une séquence de contrôle de l'expression dans la molécule d'ADN recòmbinant

5. Procédé selon la revendication 2, caractérisé en ce que la séquence d'ADN code pour l'expression de l'amino- Δ -3 Interferon gamma (IFN-g).

6. Procédé selon la revendication 5, caractérisé en ce qu'un codon de démarrage ATG est attaché directement à l'extrémité en 5' de la séquence d'ADN.

7. Procédé selon la revendication 2, caractérisé en ce que la séquence d'ADN code pour l'expression pour un polypeptide du type IFN- $\gamma$ et comporte la région qui ne code pas en 3' du gène de l'IFN- $\gamma$ et une queue de poly G en 3', la séquence d'ADN ayant une suppression de nucléotides d'au moins une portion de la région ne codant en 3', mais maintenant la queue de poly G à l'extrémité terminale carboxylique de la région ne codant pas du gène de l'IFN-$\gamma$.

8. Procédé selon la revendication 7, caractérisé en ce que la séquence d'ADN comporte la suppression de 422 nucléotides dans la région ne codant pas en 3' du gène de l'INF- $\gamma$, la suppression partant du site de reconnaissance de l'endonuclease Rsal et continuant vers la région qui code du gène de l'IFN-$\gamma$.

9. Procédé selon la revendication 7, caractérisé en ce que la séquence d'ADN comporte la suppression de 484 nucléotides dans la région ne codant pas en 3' du gène de l'INF- $\gamma$, la suppression partant du site de reconnaissance de l'endonuclease Rsal et continuant vers la région qui code du gène de l'IFN- $\gamma$.

10. Procédé selon la revendication 2, caractérisé en ce que la séquence d'ADN code pour l'expression pour IFN- $\gamma$.

11. Procédé selon la revendication 10, caractérisé en ce qu'un cordon de démarrage ATG est attaché directement à l'extrémité en 5' de séquence d'ADN.

12. Procédé selon la revendication 10, caractérisé en ce que la séquence d'ADN comporte la suppression de 422 nucléotides dans la région qui ne code pas en 3' du gène de l'IFN-$\gamma$, la suppresion partant du site de reconnaissance de l'endonuclease Rsal et continuant vers la région qui code du gène de l' INF- $\gamma$.

13. Procédé selon la revendication 10, caractérisé en ce que la séquence comporte la suppression de 484 nucléotides dans la région ne codant pas en 3' du gène de l'INF- $\gamma$, la suppression partant du site de reconnaissance de l'endonuclease Rsal et continuant vers la région qui code du gène de l'INF-$\gamma$.

14. Procédé selon l'une des revendication s5 à 13, caractérisé en ce que la séquence d'ADN est reliée opérativement à une séquence de contrôle de l'expression.

15. Cellule hôte transformée par au moins une molécule d'ADN recombinant, caractérisée en ce qu'elle comprend une séquence d'ADN préparée conformément au procédé de l'une des revendications 5 à 13.

**Ansprüche**

1. Verfahren zur Verbesserung der Expressionshöhe einer ein ausgewähltes Protein oder Polypepid codierenden und einen 3'-poly G-Schwanz tragenden DNA-Sequenz in einer Wirtszelle, die mit einem diese DNA-Sequenz enthaltenden rekombinanten DNA-Molekül tranformiert ist, wobei mindestens ein Teil des 3'-nicht-codierenden Bereichs der bei ihrer Expression das Protein oder Poleptid codierenden DNA-Sequenz deletiert, der poly G-Schwanz jedoch beibehalten wird, der transformierte Wirt gezüchtet und ˜das Protein oder Polypeptid gesammelt wird.

2. DNA-Sequenz, die bei ihrer Expression ein Protein oder Polypeptid codiert und die den 3'-nicht-codierenden Bereich des Gens für das Protein oder Polypeptid und einen 3'-poly G-Schwanz enthält, wobei die DNA-Sequenz eine Nucleotiddeletion von mindestens einem Teil des 3'-nicht-codierenden Bereichs aufweist, den poly G-Schwanz am Carboxyterminus des nicht-codierenden Bereichs jedoch beibehält, was zu einer erhöhten Expression der DNA-Sequenz führt.

3. Rekombinantes DNA-Molekül, gekennzeichnet durch die DNA-Sequenz nach Anspruch 2, wobei die DNA-Sequenz funktionell mit einer Expressionskontrollsequenz in dem rekombinanten DNA-Molekül verbunden ist.

4. Wirtszelle, transformiert mit mindestens einem rekombinanten DNA-Molekül nach Anspruch 3.

5. DNA-Sequenz nach Anspruch 2, die bei ihrer Expression Amino-Δ3-IFN-γ codiert.

6. DNA-Sequenz nach nach Anspruch 5, bei der ein ATG-Startcodon unmittelbar an das 5'-Ende angeheftet ist.

7. DNA-Sequenz nach Anspruch 2, die bei ihrer Expression ein IFN-γ-ähnliches Polypeptid codiert und den 3'-nichtcodierenden Bereich des IFN-γ-Gens und einen 3'-poly G-Schwanz enthält und wobei die DNA-Sequenz eine Nucleotiddeletion von mindestens einem Teil des 3'-nicht-codierenden Bereichs aufweist, den poly G-Schwanz am Carboryterminus des nicht-codieronden Bereichs des IFN-γ-Gens jedoch beibehält.

8. DNA-Sequenz nach Anspruch 7, wobei die DNA-Sequenz eine Deletion von 422 Nucleotiden im 3'-nicht-codierenden Bereich des IFN-γ-Gens aufweist, und diese Deletion an der RSa I Endonucleasen-Erkennungsstelle beginnt und sich in Richtung des codierenden Bereichs das IFN-γ-Gens fortsetzt.

9. DNA-Sequenz nach Anspruch 7, wobei die DNA-Sequenz eine Deletion von 484 Nucleotiden im 3'-nicht-codierenden Bereich des IFN-γ-Gens aufweist und die Deletion an der Rsa I Endonucleasen-Erkennungsstelle beginnt und sich in Richtung des codierenden Bereichs des IFN-γ-Gens fortsetzt.

10. DNA-Sequenz nach Anspruch 2, die bei ihrer Expression IFN-γ codiert.

11. DNA-Sequenz nach Anspruch 10, bei der das ATG-Startcodon unmittelbar an das 5'-Ende angeheftet ist.

12. DNA-Sequenz nach Anspruch 10, wobei die DNA-Sequenz eine Deletion von 422 Nucleotiden im 3'-nicht-codierenden Bereich des IFN-γ-Gens aufweist und die Deletion an der Rsa I-Endonucleasen-Erkennungsstelle beginnt und sich in Richtung des codierenden Bereichs des IFN-γ-Gens fortsetzt.

13. DNA-Sequenz nach Anspruch 10, wobei die DNA-Sequenz eine Deletion von 484 Nucleotiden im 3'-nicht-codierenden Bereich des IFN-γ-Gens aufweist und die Deletion an der Rsa I Endonucleasen-Erkennungsstelle beginnt und sich in Richtung des codierenden Bereichs des IFN-γ-Gens fortsetzt.

14. Rekombinantes DNA-Molekül, gekennzeichnet durch eine DNA-Sequenz, die eine DNA-Sequenz nach einem der Ansprüche 5 bis 13 ist, wobei die DNA-Sequenz in dem rekombinanten DNA-Molekül funktionell mit einer Expressionskontrollsequenz verbunden ist.

15. Wirtszelle transformiert mit mindestens einem rekombinanten DNA-Molekül nach Anspruch 14.

Patentansprüche für folgenden Vertragsstaaten: AT

1. Verfahren zur Verbesserung der Expressionshöhe einer ein ausgewähltes Protein oder Polypeptid codierenden und einen 3'-poly G-Schwanz tragenden DNA-Sequenz in einer Wirtszelle, die mit einem diese DNA-Sequenz enthaltenden rekombinanten DNA-Molekül transformiert ist, wobei mindestens ein Teil des 3'-nicht-codierenden Bereichs der bei ihrer Expression das Protein oder Polypeptid codierenden DNA-Sequenz deletiert, der poly G-Schwanz jedoch beibehalten wird, der transformierte Wirt gezüchtet und das Protein oder Polypeptid gesammelt wird.

2. Verfahren, bei dem man eine bei ihrer Expression ein Protein oder Polypeptid codierende DNA herstellt, die den 3'-nicht-codierenden Bereich des Gens für das Protein oder Polypeptid und einen 3'-poly G-Schwanz enthält, wobei die DNA-Sequenz eine Nucleotiddeletion von mindestens einem Teil des 3'-nicht-codierenden Bereichs aufweist, den poly G-Schwanz am Carboxyterminus des nicht-codierenden Bereichs jedoch beibehält, was zu einer erhöhten Expression der DNA-Sequenz führt.

3. Verfahren, bei dem man ein rekombinantes DNA-Molekül herstellt, enthaltend eine bei ihrer Expression ein Protein oder Polypeptid codierende DNA-Sequenz, die den 3'-nicht-codierenden Bereich das Gens für das Protein oder Polypeptid und einen 3'-poly G-Schwanz enthält, wobei die DNA-Sequenz eine Nucleotiddeletion von mindestens einem Teil des 3'-nicht-codierenden Bereichs aufweist, den poly G-Schwanz am Carboxyterminus des nicht-codierenden Bereichs jedoch beibehält, was zu einer erhöhten Expression der DNA-Sequenz führt und wobei die DNA-Sequenz ferner funktionell mit einer Expressionskontrollsequenz im rekombinanten DNA-Molekül verbunden ist.

4. Wirtszelle, die mit mindestens einem rekombinanten DNA-Molekül transformiert ist, enthaltend eine bei ihrer Expression ein Protein oder Polypeptid codierende DNA-Sequenz, die den 3'-nicht-codierenden Bereich des Gens für das Protein oder Polypeptid und einen 3'-poly G-Schwanz enthält, wobei die DNA-Sequenz eine Nucleotiddeletion von mindestens einem Teil des 3'-nicht-codierenden Bereichs aufweist, den poly G-Schwanz am carboxyterminus des nicht-codierenden Bereichs jedoch beibehält, was zu einer erhöhten Expression der DNA-Sequenz führt und wobei die DNA-Sequenz ferner funktionell mit einer Expressionskontrollsequenz im rekombinanten DNA-Molekül verbunden ist.

5. Verfahren nach Anspruch 2, wobei die DNA-

Sequenz Amino-Δ3-IFN-γ codiert

6. Verfahren nach Anspruch 5, wobei ein ATG-Startcodon unmittelbar an das 5'-Ende der DNA-Sequenz angeheftet ist.

7. Verfahren nach Anspruch 2, wobei die DNA-Sequenz bei ihrer Expression ein IFN-γ-ähnliches Polypeptid codiert und den 3'-nicht-codierenden Bereich des IFN-γ-Gens und einen 3'-poly G-Schwanz enthält und wobei die DNA-Sequenz eine Nucleotiddeletion von mindestens einem Teil des 3'-nicht-codierenden Bereichs aufweist, den poly G-Schwanz am Carboxyterminus des nicht-codierenden Bereichs des IFN-γ-Gens jedoch beibehält.

8. Verfahren nach Anspruch 7, wobei die DNA-Sequenz eine Deletion von 422 Nucleotiden im 3'-nicht-codierenden Bereich des IFN-γ-Gens aufweist, und diese Deletion an der Rsa I Endonucleasen-Erkennungsstelle beginnt und sich in Richtung des codierenden Bereichs des IFN-γ-Gens fortsetzt.

9. Verfahren nach Anspruch 7, wobei die DNA-Sequenz eine Deletion von 484 Nucleotiden im 3'-nicht-codierenden Bereich das IFN-γ-Gens aufweist und die Deletion an dar Rsa I Endonucleasen-Erkennungsstelle beginnt und sich in Richtung des codierenden Bereichs des IFN-γ-Gens fortsetzt.

10. Verfahren nach Anspruch 2, wobei die DNA-Sequenz bei ihrer Expression IFN-γ codiert.

11. Verfahren nach Anspruch 10, wobei ein ATG-Startcodon unmittelbar an das 5'-Ende der DNA-Sequenz angeheftet ist.

12. Verfahren nach Anspruch 10, wobei die DNA-Sequenz eine Deletion von 422 Nucleotiden im 3'-nicht-codierenden Bereich des IFN-γ-Gens aufweist und diese Deletion an der RSa I-Endonucleasen-Erkennungsstelle beginnt und sich in Richtung des codierenden Bereichs das IFN-γ-Gens fortsetzt.

13. Verfahren nach Anspruch 10, wobei die DNA-Sequenz eine Deletion von 484 Nucleotiden im 3'-nicht-codierenden Bereich des IFN-γ-Gens aufweist und. die Deletion an der Rsa I Endonucleasen-Erkennungsstelle beginnt und sich in Richtung des codierenden Bereichs des IFN-γ-Gens fortsetzt.

14. Verfahren nach einem der Ansprüche 5 bis 13, wobei die DNA-Sequenz funktionell mit einer Expressionskontrollsequenz verbunden ist.

15. Wirtszelle transformiert mit mindestens einem rekombinanten DNA-Molekül, enthaltend eine nach dem Verfahren nach einem der Ansprüche 5 bis 13 hergestellte DNA-Sequenz.

FIG.1

*FIG.2*

FIG.2.

ClaI

Xho I site

~~trp~~ AAGGGTATCGATACTATG [ IFN-ɣ (mature) coding-region | IFN-ɣ non-coding ] CCCATGCCTCGAGG—

Shine Dalgarno

IFN-ɣ (mature) coding-region

IFN-ɣ non-coding

RsaI site

BamHI site

CATG GGT GTAC GGATCC ~~

IFN-ɣ non coding region

*FIG. 3.*

EP 0 174 999 B1

start codon
for
transcription

Cla I site

double-stranded
plasmid
DNA sequence

C G A T A C T A T G T G T T A C T G C C A G G A C C C

G C T A T G A T A C A C A A T G A C G G T C C T G G G

prime sequence   C G A T A C T A T G————△————C A G G A C C C

9 nucleotide

FIG.4.

```
              T
            A   G
            A   A
            C   C
     3'     A   G        5'
hybridization  G C T A T G A T A C G T C C T G G G  ◄——— plasmid DNA

        5' C G A T A C T A T G C A G G A C C C 3'  ◄——— primer DNA
```

## FIG.5.

### SEQUENCE OF DNA INSERT

```
      RI                    Hinf                                      Hha-Pvu   PvwII              SauSA              Fnu
      |                     |    20                        40              |60      |         80       |         100    |
   pBR-GAATTCGGCAATAAGATTCAACGCCAGTCCCGAACGTGAAATTTCCTCTCTTGCTGGCGCGATTGCAGCTGTGGTGTCATGGTCGGTGATCGCCAGGGTGCCGACGC

   Hha  Tag                                                               ALu
   |    |     120               140                                       |160              180                200
   GCATCTCGACTGCACGGTGCACCATTGCTTCTGGCGTCAGGCAGCCATCGGAAGCTGTGGTATGGCTGTGCAGGTCGTAAATCACTGCATAATTCGTGTCGTCCAAGGCGC

                         Hha                                           Alu  Hine          Taq        HpaI   RSA
      220               |              260                      280    |    |        300  |          |      320|   ┌──────m.RNr
   ACTCCCGTTCTGGATAATGTTTTTTGCGCCGACATCATAACGGTTCTGGCAAATATTCTGAAATGAGCTGTTGACAATTAATCATCGAACTAGTTAACTAGTACGCAAGTT
                     cla                                                      ‾‾‾‾‾                 ‾‾‾‾‾‾
                                                                              c.s.                  p.g

      340
   CACGTAAAAAGGGTATCGATACT ATG TGT TAC TGC CAG GAC CCA TAT GTA AAA GAA GCA GAA AAC CTT AAG AAA TAT TTT AAT GCA GGT
     ‾‾‾‾ SD

   CAT TCA GAT GTA GCG GAT AAT GGA ACT CTT TTC TTA GGC ATT TTG AAG AAT TGG AAA GAG GAG AGT GAC AGA AAA ATA ATG CAG

   AGC CAA ATT GTC TCC TTT TAC TTC AAA CTT TTT AAA AAC TTT AAA GAT GAC CAG AGC ATC CAA AAG AGT GTG GAG ACC ATC AAG

   GAA GAC ATG AAT GTC AAG TTT TTC AAT AGC AAC AAA AAG AAA CGA GAT GAC TTC GAA AAG CTG ACT AAT TAT TCG GTA ACT GAC

   TTG AAT GTC CAA CGC AAA GCA ATA CAT GAA CTC ATC CAA GTG ATG GCT GAA CTG TCG CCA GCA GCT AAA ACA GGG AAG CGA AAA

   AGG AGT CAG ATG CTG TTT CGA GGT CGA AGA GCA TCC CAG TAA TGGTTGTCCTGCCTGCAATATTTGAATTTTAAATCTAAATCTATTTATTAATATT

   TAACATTATTTATATGGGGAATATATTTTTAGACTCATCAATCAAATAAGTATTTATAATAGCAACTTTTGTGTAATGAAAATGAATATCTATTAATATATGTATTATTTA

   TAATTCCTATATCCTGTGACTGTCTCACTTAATCCTTTGTTTTCTGACTAATTAGGCAAGGCTATGTGATTACAAGGCTTTATCTCAGGGGCCAACTAGGCAGCCAACCTA

                  XhoI
                  |
   AGCAAGATCCCATGCCTCGAGGCATGGTTGTGTGTTTATTTCACTTGATGATACAATGAACACTTATAAGTGAAGTGATACTATCCAGTTACTGCCGGTTTGAAAATATGC

   CTGCAATCTGAGCCAGTGCTTTAATGGCATGTCAGACAGAACTTGAATGTGTCAGGTGACCCTGATGAAAACATAGCATCTCAGGAGATTTCATGCCTGGTGCTTCCAAAT
        Hine              RSAI                              BamHI
        |                 |                                 |
   ATTGTTGACAACTGTGACTGTACCCAAGGGGGGGGGGGGGGGGGGAGGGGGGGGATCC-pBR
```